Europäisches Patentamt

European Patent Office

Office européen des brevets

(19)

(11) Publication number: **0 190 790**
**B1**

# EUROPEAN PATENT SPECIFICATION

(12)

(45) Date of publication of the patent specification:
26.07.89

(51) Int. Cl.⁴: **C07C 37/70**, C07C 39/04,
C07C 37/08

(21) Application number: **86200117.9**

(22) Date of filing: **29.01.86**

(54) **Process for the purification of phenol.**

(30) Priority: **08.02.85 IT 1945185**

(43) Date of publication of application:
**13.08.86 Bulletin 86/33**

(45) Publication of the grant of the patent:
**26.07.89 Bulletin 89/30**

(84) Designated Contracting States:
**AT BE CH DE FR GB LI LU NL SE**

(56) References cited:
**EP-A- 0 004 168**
**AT-B- 205 489**
**AT-B- 205 490**
**DE-A- 2 211 616**
**GB-A- 920 905**
**GB-A- 1 021 759**
**GB-A- 1 108 327**
**GB-A- 1 171 854**
**US-A- 2 971 893**
**US-A- 3 454 653**
**US-A- 3 965 187**

**Römpps Chemie-Lexikon, 8th ed., pp. 2623-2624**

**The file contains technical information submitted after the application was filed and not included in this specification**

(73) Proprietor: **ENICHEM ANIC S.p.A., Via Ruggero Settimo 55, I-90139 Palermo(IT)**

(72) Inventor: **Messina, Giuseppe, Via Perpignan 27, I-07041 Alghero, Sassari(IT)**
Inventor: **Bruzzi, Vittorio, Via Assietta 31, I-20161 Milan(IT)**
Inventor: **Lorenzoni, Loreno, Via Sassari 184, I-07046 Porto Torres, Sassari(IT)**
Inventor: **Voltolini, Maurizio, Via Mecenate 7, I-20138 Milan(IT)**

(74) Representative: **Roggero, Sergio et al, Ing. Barzanò & Zanardo Milano S.p.A. Via Borgonuovo 10, I-20121 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for purifying a stream of raw phenol exiting a process of preparation of phenol and acetone from cumene, via cumene hydroperoxide.

The prior art teaches to produce phenol by oxidizing cumene to cumene hydroperoxide and then splitting the latter in the presence of acidic catalysts. The phenol thus obtained, however, has acetone as a main impurity and contains other by-products and, even when it is purified by fractional distillation, is unsuitable for preparing certain compounds, such as bisphenol, polycarbonates, polyamides: for preparing these products a very high purity phenol is required and such a phenol can be obtained with difficulty by distillation and rectification.

Phenol purity tests are generally colorimetric assays which follow nitration, chlorination, or sulphonation, which are apt to induce discolourations indicative of chromogenic impurities. Therefore, a high purity phenol is one which does not originate discolourations under the conditions aforesaid.

For these reasons, phenol is conventionally purified by multiple fractional distillations, and particular treatments have been suggested by the prior art for removing the chromogenic impurities.

With this objective in view, the prior art offers treatments with acidic condensing agents (DE-A 2 211 616), high-temperature treatments (over 220°C) with acidic silicoaluminates such as in US-A 3 454 653, treatments with alkalies and ferric chloride, such as in GB-A 920 905, the use of polyamines (US-A 3 965 187), and the use of hydrogen peroxide in an alkaline medium, suggested by US-A 2 971 893.

All of these treatments are objectionable due to their cost, their intricacy, and the risk of polluting the substance to be purified, and, above all, bcause they do not solve the purification problem satisfactorily.

Distillation processes have also been proposed, such as GB-A 1 021 759 and EP-A 0 004 168: these latter are difficult to work and are not quite satisfactory in removing chromogenic impurities.

More particularly, US-A 3 454 653 discloses the purification of impure phenol from cumene, at a high temperature, with a silica-alumina catalyst and under substantially anhydrous conditions: to obtain fair results, however, the reference teaches to use a chromium-modified catalyst and high temperatures (200°C to 240°C). Apart from the fact that such a modified catalyst is expensive, the common commercial acidic earths cannot be used as they become rapidly fouled and unusable, when put to work at so high temperatures.

GB-A 1 108 327 and GB-A 1 171 854 have in common high temperature steam treatments whereby hydroxyacetone is porevailingly converted into 2-methylbenzofuran, to be removed by azeotropic distillation together with the water contained in the raw phenol stream.

To conclude the review of the prior art, AT-B 205 490 combines distillation runs and treatments with alkaline agents.

The technical problem of preparing a high purity phenol without suffering from the drawbacks and limitations of the prior art enumerated and discussed above, is solved, according to the present invention, by a process for purifying a stream of raw phenol exiting a process of preparation of phenol and acetone from cumene, via cumene hydroperoxide, comprising the steps of:

– feeding the raw phenol stream, containing alpha-methyl-styrene, from 2% to 4% by weight water, cumene, mesityl oxide, hydroxyacetone and other impurities, to an intermediate point between the head and the bottom of a first distillation column equipped with a reboiler, a condenser and a reflux;

– seperating within said first column a bottom stream composed of phenol and alpha-methyl-styrene, from a head stream composed of alpha-methyl-styrene, water, cumene and hydroxyacetone;

– condensing said head stream to form an aqueous phase and an organic phase, a part of said organic phase being recycled to the column head;

– feeding the bottom stream from said first column in the vicinity of the head of a second distillation column which is equipped with a reboiler and which is free from condenser and from reflux;

– separating the bottom stream exiting said second column, essentially consisting of phenol from the head stream exiting said second column, essentially consisting of alpha-methyl-styrene and phenol;

– recycling said second column head stream directly in the vicinity of the bottom of said first column;

– contacting the bottom stream exiting said second column, at a temperature between 120°C and 200°C, for a time between 10 min and 4 h, and under a pressure between atmospheric (101,325 kPa) and 490,33 kPa (5 kg/cm²), with a natural silicoaluminate, having a silica/alumina weight ratio of at least 3:1, and which has been pretreated with a mineral acid so as to reduce its alkali metal content to less than 0,1% by weight;

– feeding the stream exiting the silicoaluminate treatment to a point intermediate between the head and the bottom of a third distillation column, and separating:

– a head fraction consisting of phenol and light impurities;

– a bottom fraction consisting of phenol and heavy impurities, and

– a side fraction of purified phenol.

Said purified phenol shows a total content of carbonylic compounds lower than 50 ppm, and is practically colourless at sulphonation test.

According to the present invention the product of acidic splitting of cumene hydroperoxide, after the neutralization of catalyst, is submitted to distillation treatments to seperate the light products, in particular acetone and a portion of hydrocarbons, such as cumene and alpha-methyl-styrene and the heavy products, such as acetophenone, alpha-methyl-styrene dimers and pitches.

The stream is thus obtained of raw phenol, which is the feed to the process of the present invention, and which typically contains cumene within the range of from 0.1 to 1% by weight, alpha-methyl-styrene within the range of from 2 to 10% by weight, water within the range of from 2 to 4% by weight, mesityl oxide within the range of 100–400 ppm, hydroxyacetone within the range of 100–2000 ppm and many other impurities among which phenylbutenes, aliphatic ketones with 9 carbon atoms in their molecule, and acetic acid. According to the present invention, such a stream of raw phenol is fed into an intermediate point between the head and the bottom of the first distillation column, provided with reboiler, condenser and reflux, and operating under a pressure lower than atmospheric pressure.

Typically, this first distillation column contains 40 trays, and the feed occurs between the 20th and the 30th tray. Moreover, it is typically run under a pressure measured at the head of the order of (159,6 mber mmHg), with a bottom temperature of the order of 100°C, with a bottom temperature of the order of 130°C, and with a reflux ratio of 25–30.

In particular, the organic phase is refluxed, as it shall be illustrated hereinunder.

By operating under these conditions, at the head of the column a vapour stream, essentially constituted by alpha-methyl-styrene (40–90% by weight), water (0,5–5% by weight), cumene, the most of hydroxycetone present in the feed, and variable amounts of other impurities and of phenol, in proper amounts due to the azeotropes with alpha-methyl-styrene and cumene, is separated.

This vapour stream is condensed and separated into an aqueous phase (containing the most of hydroxyacetone, a little of phenol and other impurities) and into an organic phase (which is essentially constituted by alpha-methyl-styrene, cumene, phenol and traces of hydroxyacetone). The aqueous phase is removed from the system, and a portion of the organic phase constitutes the reflux to the distillation column, the balance is recovered.

It should be noted that the presence of water has a beneficial function, in that hydroxyacetone and the other impurities are separated in the aqueous phase, thus avoiding on one hand the presence of hydroxyacetone in the reflux, and on the other hand, the presence of hydroxyacetone in the organic phase separated at the head, and intended for recycle.

Thus, the stream of raw phenol fed to the first distillation column must contain an amount of water not lower than about 2% by weight, to achieve the above-described effects. Conveniently, the maximum concentration of water inside said stream of raw phenol does not exceed about 4% by weight, in that beyond such a value no appreciable advantages are obtained.

This water can be present in the stream of raw phenol outcoming from previous distillation treatments, or it must be added to the same stream, before that this may be submitted to the process according to the present invention.

By operating as previously described, at the bottom of the first distillation column a liquid stream is separated, essentially constituted by phenol and by alpha-methyl-styrene, with a content of alpha-methyl-styrene of from 30 to 50% by weight, and preferably at a value close to the high limit of said range, in that it allows a better separation of the impurities.

The bottom stream from the first distillation column is fed into the nearby of the head of a second distillation column, provided with reboiler, but free from condenser and reflux.

Typically, this second distillation column contains 90 trays, and the feeding takes place in correspondence of the 90th tray.

This column is moreover typically run under a pressure measured at the head of the order of 452,2 mbar (340 mmHg), with a head temperature of the order of 137°C, and with a bottom temperature of the order of 175°C.

By operating under these conditions, at the head of the second column a vapour stream of alpha-methyl-styrene and phenol, containing small amounts of impurities, is separated.

This stream is directly recycled to the bottom of the first distillation column. Moreover, at the bottom of the second column a liquid stream essentially constituted by phenol (purity higher than about 99.5% by weight) and with a content of carbonylic compounds generally ranging from 200 to 400 ppm is separated. In this distillation, reducing the amount of carbonylic compounds, such as hydroxyacetone, to very low values, e.g., to values lower than about 50 - 70 ppm, is not necessary.

The reduction of these carbonylic compounds under such levels can be indeed carried out, according to the present invention, in the subsequent treatments, whose function is of transforming and eliminating in a substantially complete way both the impurities of carbonylic character and non-carbonylic impurities.

More particularly, according to the present invention, the phenol liquid stream separated at the bottom of the second distillation column is placed in contact with a natural silico-aluminate, having a silica/alumina weight ratio equal to or greater than 3/1, previously treated with mineral acid.

Natural silico-aluminates suitable to that purpose are bentonites, in particular those showing a high content of montmorillonite. It is known that montmorillonite is a silico-aluminate of sodium, calcium and magnesium, whose general formula is as follows:

$$[Al_{1.67}Mg_{0.33}Na_{0.33}]Si_4O_{10}(OH)_2$$

The concentration of montmorillonite inside bentonites is generally ranging from 60 to 80% by weight, the balance being formed by such materials as silica, calcite and illite. By treating these silico-aluminates with mineral acids, such as sulphuric acid, hydrofluoric acid and hydrochloric acid, the alkaline metals are totally or partly removed and replaced by hydrogen, and the so-called "activated acidic earths" are obtained.

To the purposes of the present invention, those natural silico-aluminates are useful, which show a silica/alumina weight ratio equal to or greater than 3/1, and which have been pre-treated with a mineral acid, up to remove the alkaline metals, or at least up to reduce them to a content lower than about 0.1% by weight.

A product of this type is commercially available, under the trade name Filtrol G-24, from the firm Filtrol Co., of Los Angeles (USA).

Thus, according to the present invention, the contact between the liquid stream of phenol outcoming from the second distillation column and the silico-aluminate having the characteristics as above described, is carried out at a temperature of from 120 to 200°C, from a time of from 10 minutes to 4 hours and preferably of from 0.5 to 2 hours, under a pressure of from atmospheric pressure to about 5 kg/cm².

Conveniently, the silico-aluminate with granulometry of from 10 to 100 mesh, is given the form of a fixed bed in a reactor, at whose bottom the liquid stream of phenol to be processed is fed.

The silico-aluminate having the characteristics as previously described, shows the advantage, compared to other silico-aluminates of the known art, of being active within a range of quite low temperatures.

Relatively to the sulphonic resins, used to the same purpose, the silico-aluminates according to the present invention yield a series of advantages, such as the possibility of operating within such a temperature range as not to require the cooling of the phenol liquid stream outcoming from the second distillation column, the lower cost, the absence of any types of activation and regeneration steps, and the absence of acidic residues inside treated phenol.

The treatment with silico-aluminate according to the present invention allows converting mesityl oxide, hydroxyacetone, and any other carbonylic or olephinic impurities present into lighter or heavier compounds, which can be easily separated from phenol in the subsequent rectification step.

Hence, according to the present invention, the liquid stream of phenol treated over the silico-aluminate is fed into a point intermediate between the head and the bottom of a distillation column provided with reboiler, condenser and reflux, typically containing 50 trays, the feeding taking place in correspondence of the 18th tray.

This column is typically run under a pressure measured at the head of the order of 100 mmHg, with a head temperature of the order of 120°C, with a bottom temperature of the order of 159°C, and with a reflux ratio of the order of about 1.

Under these conditions, at the head of the column a stream of phenol vapour with light impurities, and at the bottom a liquid stream of phenol with heavy impurities is separated. These two phenol streams are recycled.

Purified phenol is drawn from the column as side stream (preferably in correspondence of the 45th tray).

At the chemical analysis, this purified phenol shows a content of carbonylic compounds lower than 50 ppm,

Moreover, this purified phenol is submitted to the following sulphonation test. To 20 ml of purified phenol, maintained under vacuum, at 45°C and under stirring for 10 minutes, 20 ml of high-purity concentrated (96%) sulphuric acid are added.

The mixture is left standing for 4 minutes, the stirring being so adjusted as to prevent the formation of air bubbles. The vacuum is then withdrawn, and the mass is cooled for 5 minutes at 20°C. The measurement of transmittance at 532 nm is immediately carried out, inside 10-mm cells, using demineralized water, whose transmittance is posed equal to 100, as the reference. Phenol purified according to the process according to the present invention shows transmittance values, at sulphonation test, similar to those of water.

In the following experimental example, the parts and percentages are to be intended as being by weight, if not otherwise specified.

Example

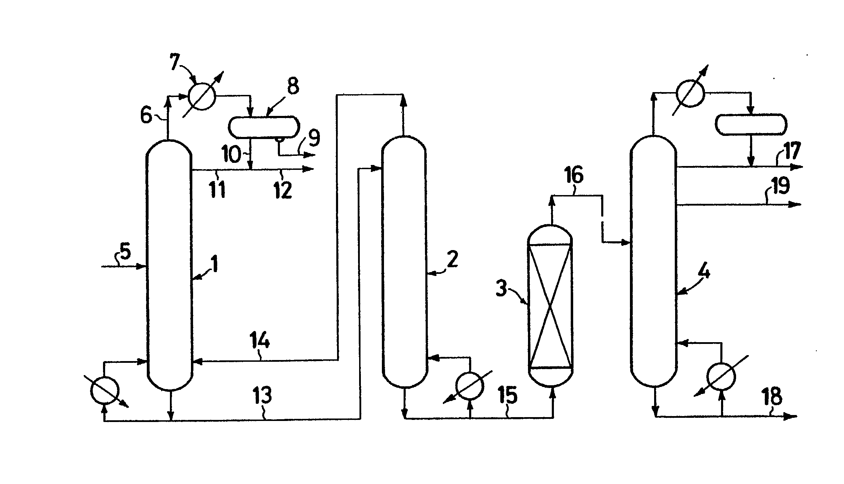

Referring the figure of attached drawing table, to the distillation column 1, through the line 5, a liquid stream is fed of raw phenol (1570 parts/hour) heated at the temperature of about 70°C, and having the following composition:

| — cumene | 0.19% |
| — alpha-methyl-styrene | 4.1% |
| — phenol | 92.71% |
| — acetophenone | 0.1% |
| — water | 2.56% |
| — hydroxyacetone | 0.17% |
| — mesityl oxide | 220 ppm |
| — heavy products | 0.15% |

Column 1 is a 40-tray distillation column, provided with reboiler, condenser and reflux. The feeding (line 5) takes place in correspondence of the 26th tray of column 1, and the column is run under the following conditions: head pressure 159,6 mbar (120 mmHg), head temperature 100°C, bottom temperature 130°C and reflux ratio 29.

Under these conditions, at the head of column 1 (line 6) 114 parts/hour are drawn of a distillate which is coolled in exchanger 7 and is separated, in decanter 8, in an organic phase containing cumene, alpha-methyl-styrene and phenol (71 parts) and in an aqueous phase (43 parts) containing 6.2% of hydroxyacetone, in addition to small amounts of phenol.

The aqueous phase is recovered (line 9) and the organic phase, drawn through the line 10, is partly sent to column 1 as reflux (line 11), and the balance is recovered (line 12).

By operating under the above indicated conditions, from the column a bottom product is recovered, having the following composition:

| — alpha-methyl-styrene | 48.92% |
| — phenol | 51.0% |
| — hydroxyacetone | 75 ppm |
| — mesityl oxide | 65 ppm |
| — heavy products | 0.04% |
| — acetophenone | 290 ppm |

This bottom product is sent (line 13) to the head tray of a distillation column 2, of 90 trays, provided with reboiler and operating under the following conditions: head pressure 452,2 mbar (340 mmHg), head temperature 137°C, bottom temperature 175°C.

Under these conditions, at the head of column 2 a distillate is drawn, which is directly sent (line 14) to the bottom of column 1, whilst at the bottom of column 2, a liquid stream (1456 parts/hour) is drawn (line 15), having the following compositions:

| – phenol | 99.70% |
|---|---|
| – hydroxyacetone | 35 ppm |
| – mesityl oxide | 260 ppm |
| – acetophenone | 0.11% |
| – heavy products | 1650 ppm |

This liquid stream is fed (line 15) to the bottom of reactor 3, packed with a silico-aluminate having the following composition:

| $SiO_2$ | 67.2% |
|---|---|
| $Al_2O_3$ | 13.8% |
| $Fe_2O_3$ | 0.99% |
| Ca | 0.82% |
| Na | 0.02% |
| $H_2O$ | balance to 100 |

in the form of pellets of from 30 to 60 mesh and known under the trade name FILTROL G-24, available from Filtrol Co. of Los Angeles.

Inside reactor 3, the process is carried out at 170°C, under a pressure of 392,264 kPa (4 kg/cm²) and with a permanence time of about 1 hour, and a liquid stream is recovered (line 16), which is fed, in correspondence of the 18th tray, into the distillation column 4, equipped with 50 trays, provided with reboiler, condenser and reflux. Column 4 is run under a head pressure of 133,3 mbar (100 mmHg), with a head temperature of 120°C, with a bottom temperature of 159°C, and with a reflux ratio around 1.

Under these conditions, at the head of the column (line 17) a phenol stream containing light impurities is separated, and at the bottom of the column (line 18) a stream of phenol containing heavy impurities is separated. Sideways to column 4, in correspondence of the 45th tray, a stream of purified phenol (960 parts/hour) is drawn (line 19).

The streams in (17) and (18) are recycled to the process, the stream (19) is recovered and submitted to the analysis. The chemical analysis shows a content of carbonylic compounds lower than 50 ppm. The sulphonation test shows a value of transmittance at 532 nm of 96.5.

When operating in the way as previously indicated, but omitting the treatment over silico-aluminate in reactor 3, a stream is obtained of purified phenol (line 19) with a content of carbonylic compounds of about 300 ppm which, at the sulphonation test, shows a value of transmittance at 532 nm of 87.5.

## Claims

1. Process for purifying a stream of raw phenol exiting a process of preparation of phenol and acetone from cumene, via cumene hydroperoxide, comprising the steps of:
– feeding the raw phenol stream, containing alpha-methyl-styrene, from 2% to 4% by weight water, cumene, mesityl oxide, hydroxyacetone and other impurities, to an intermediate point between the head and the bottom of a first distillation column equipped with a reboiler, a condenser and a reflux;
– separating within said first column a bottom stream composed of phenol and alpha-methyl-styrene, from a head stream composed of alpha-methyl-styrene, water, cumene and hydroxyacetone;
– condensing said head stream to form an aqueous phase and an organic phase, a part of said organic phase being recycled to the column head;
– feeding the bottom stream from said first column in the vicinity of the head of a second distillation column which is equipped with a reboiler, and which is free from condenser and from reflux;
– separating the bottom stream exiting said second column, essentially consisting of phenol, from the head stream exiting said second column, essentially consisting of alpha-methyl-styrene and phenol;
– recycling said second column head stream directly in the vicinity of the bottom of said first column;
– contacting the bottom stream exiting said second column, at a temperature between 120°C and 200°C, for a time between 10 min and 4 h, and under a pressure between atmospheric (101,325 kPa) and 490,33 kPa (5 kg/cm²), with a natural silicoaluminate, having a silica/alumina weight ratio of at least 3:1, and which has been pretreated with a mineral acid so as to reduce its alkali metal content to less than 0,1% by weight;
– feeding the stream exiting the silicoaluminate treatment to a point intermediate between the head and the bottom of a third distillation column, and separating:
– a head fraction consisting of phenol with light impurities;
– a bottom fraction consisting of phenol and heavy impurities, and
– a side fraction of purified phenol.

2. Process according to claim 1, wherein the raw phenol fed to the first column contains from 0,1% to 1% by weight of cumene, from 2% to 10% by weight of alpha-methyl-styrene, from 2% to 4% by weight of water, from 100 ppm (parts per million) to 400 ppm of mesityl oxide, from 100 ppm to 2.000 ppm of hydroxyacetone and a balance of other impurities.

3. Process according to claim 1, wherein the first distillation column has 40 trays, the raw phenol is fed between the 20th and the 30th tray, the working pressure measured at the head of the column is 159,6 mba (120 mmHg), the head temperature is 100°C and the bottom temperature is 130°C, and the reflux ratio is 25 to 30, the bottom product of said column cosisting essentially of phenol and alpha-methyl-styrene, the latter compound representing from 30% to 50% by weight of the bottom product.

4. Process according to claim 1, wherein, in said second distillation column, the head pressure is 452,2 mbar (340 mmHg), the head temperature is 137°C, and the bottom temperature is 175°C.

5. Process according to claim 1, wherein, in the third distillation column, the head presssure is 133,3 mbar (100 mmHg), the head temperature is 120°C, the bottom temperature is 159°C, and the reflux ratio is 1.

## Patentansprüche

1. Verfahren zur Reinigung eines Stromes von Rohphenol, das aus einem Verfahren zur Herstellung von Phenol und Aceton aus Cumen oder Cumenhydroperoxid stammt, welches die folgenden Stufen umfaßt:
– Einspeisen des Rohphenolstroms, der α-Methylstyrol, 2 bis 4 Gewichts-% Wasser, Cumen, Mesityloxid, Hydroxyaceton und andere Verunreinigungen enthält, an einer intermediären Stelle zwischen dem Kopf und dem Sumpf einer ersten Destillationskolonne, die mit einem Aufkocher, einem Kühler und einem Rückfluß ausgerüstet ist;
– Abtrennen eines aus Phenol und α-Methylstyrol, gebildeten Bodenstroms innerhalb dieser ersten Kolonne von einem Kopfstrom, der aus α-Methylstyrol, Wasser, Cumen und Hydroxyaceton zusammengesetzt ist;
– Kondensieren dieses Kopfstroms zur Ausbildung einer wäßrigen Phase und einer organischen Phase, wobei ein Teil dieser organischen Phase zum Kolonnenkopf zurückgeführt wird;
– Einspeisen des Bodenstroms aus der genannten ersten Kolonne in der Nähe des Kopfes einer zweiten Destillationskolonne, die mit einem Aufkocher ausgerüstet ist und frei von einem Kühler und von Rücklauf ist;
– Abtrennen des aus dieser zweiten Kolonne austretenden, im wesentlichen aus Phenol bestehenden Bodenstromes von dem aus dieser zweiten Kolonne austretenden Kopfstrom, der im wesentlichen aus α-Methylstyrol, und Phenol besteht;
– Zurückführen dieses Kopfstromes aus der zweiten Kolonne unmittelbar in der Nähe des Bodens der genannten ersten Kolonne;
– In-Berührung-Bringen des aus dieser zweiten Kolonne austretenden Bodenstroms bei einer Temperatur zwischen 120°C und 200°C während einer Zeitdauer zwischen 10 Minuten und 4 Stunden unter einem Druck zwischen dem Atmosphärendruck (101,325 kPa) und 490,33 kPa (5 kg/cm²) mit einem natürlichen Silicoaluminat, das ein Kieselsäure/Aluminiumoxid-Gewichtsverhältnis von wenigstens 3:1 aufweist und das mit einer Mineralsäure vorbehandelt worden ist, um seinen Alkalimetallgehalt auf unter 0,1 Gewichts-% zu reduzieren;
– Einspeisen des aus der Silicoaluminatbehandlung austretenden Stroms an einem zwischen dem Kopf und dem Boden einer dritten Destillationskolonne gelegenen Punkt, und Abtrennen
– einer aus Phenol und leichten Verunreinigungen bestehenden Kopffraktion;
– einer aus Phenol und schweren Verunreinigungen bestehenden Bodenfraktion, und

– einer aus gereinigtem Phenol bestehenden Seitenfraktion.

2. Verfahren nach Anspruch 1, worin das der ersten Kolonne zugeführte Rohphenol von 0,1 bis 1 Gew.-% Cumen, von 2 bis 10 Gew.-% α-Methylstyrol, von 2 bis 4 Gewichts-% Wasser, von 100 ppm (Teile per Million) bis 400 ppm Mesityloxid, von 100 ppm bis 2.000 ppm Hydroxyaceton und eine Menge weiterer Verunreinigungen enthält.

3. Verfahren nach Anspruch 1, worin die erste Destillationskolonne 40 Böden aufweist, das Rohphenol zwischen dem 20. und dem 30. Boden eingespeist wird, der Arbeitsdruck, gemessen am Kolonnenkopf, 159,6 mbar (120 mmHg) beträgt, die Kopftemperatur 100°C und die Sumpftemperatur 130°C beträgt und das Rückflußverhältnis 25 bis 30 ausmacht, wobei das Sumpfprodukt aus dieser Kolonne im wesentlichen aus Phenol und α-Methylstyrol besteht, wobei die letztgenannte Verbindung 30 bis 50 Gewichts-% des Sumpfproduktes ausmacht.

4. Verfahren nach Anspruch 1, worin in der genannten zweiten Destillationskolonne der Kopfdruck 452,2 mbar (340 mmHg) beträgt, die Kopftemperatur 137°C beträgt und die Sumpftemperatur 175°C beträgt.

5. Verfahren nach Anspruch 1, worin in der dritten Destillationskolonne der Kopfdruck 133,3 mbar (100 mmHg) beträgt, die Kopftemperatur 120°C beträgt, die Sumpftemperatur 159°C beträgt und das Rückflußverhältnis 1 ist.

## Revendications

1. Procédé pour purifier un courant de phénol brut sortant d'un procédé de préparation de phénol et d'acétone à partir de cumène, par l'intermédiaire de l'hydropéroxyde de cumène, comprenant des étapes consistant à:
– amener le courant de phénol brut, contenant de l'âlpha-méthyl-styrène, de 2 à 4% en poids d'eau, du cumène, de l'oxyde de mésityle, de l'hydroxyacétone et d'autres impuretés, en un point intermédiaire entre la tête et le fond d'une première colonne de distillation, équipée d'un rebouilleur, d'un condenseur et d'un dispositif de reflux;
– séparer, dans ladite première colonne, un courant de fond, composé de phénol et d'alpha-méthyl-styrène, d'avec un courant de tête composé d'alpha-méthyl-styrène, d'eau, de cumène et d'hydroxyacétone;
– condenser ledit courant de tête pour former une phase aqueuse et une phase organique, une partie de ladite phase organique étant recyclée vers la tête de colonne.
– amener le courant de fond de ladite première colonne au voisinage de la tête d'une seconde colonne de distillation qui est équipée d'un rebouilleur, mais qui ne comporte pas de condenseur ni de dispositif de reflux;
– séparer le courant de fond sortant de ladite seconde colonne, constitué essentiellement de phénol, d'avec le courant de tête sortant de ladite seconde colonne, constitué essentiellement d'alpha-méthyl-styrène et de phénol;

— recycler ledit courant de tête de la seconde colonne directement au voisinage du fond de ladite première colonne;

— mettre en contact le courant de fond issu de ladite seconde colonne, à une température située entre 120 et 200°C, pendant une durée comprise entre 10 minutes et 4 heures, et sous une pression située entre la pression atmosphérique (101,325 kPa) et 490,33 kPa (5 kg/cm²), avec un alumino-silicate naturel, présentant un rapport pondéral silice/alumine d'au moins 3:1, et que l'on a traité au préalable avec un acide minéral de façon à réduire sa teneur en métal alcalin à moins de 0,1% en poids;

— amener le courant issu du traitement par l'aluminosilicato en un point intermédiaire entre la tête et le fond d'une troisième colonne de distillation, et séparer:

— une fraction de tête constituée de phénol avec des impuretés légères,

— une fraction de fond constituée de phénol et d'impuretés lourdes, et

— une fraction intermédiaire de phénol purifié.

2. Procédé conforme à la revendication 1, dans lequel le phénol brut amené à la première colonne contient de 0,1% à 1% en poids de cumène, de 2% à 10% en poids d'alpha-méthyl-styrène, de 2% à 4% en poids d'eau, de 100 ppm (parties par million) à 400 ppm d'oxyde de mésityle, de 100 ppm à 2000 ppm d'hydroxyacétone, et un complément d'autres impuretés.

3. Procédé conforme à la revendication 1, dans lequel la première colonne de distillation comporte 40 plateaux, le phénol brut est amené entre le 20ème et le 30eme plateau, la pression de travail, mesurée en tête de la colonne, est de 159,6 mbar (120 mmHg), la température de tête est de 100°C, et la température de fond est de 130°C, et le taux de reflux est 25 à 30, le produit de fond de ladite colonne étant constitué essentiellement de phénol et d'alpha-méthyl-styrène, ce dernier composé représentant de 30% à 50% en poids du produit de fond.

4. Procédé conforme à la revendication 1, dans lequel, dans ladite seconde colonne de distillation, la pression de tête vaut 452,2 mbar (340 mmHg), la température de tête vaut 137°C et la température de fond vaut 175°C.

5. Procédé conforme à la revendication 1, dans lequel, dans la troisième colonne de distillation, la pression de tête vaut 133,3 mbar (100 mmHg), la température de tête vaut 120°C, la température de fond vaut 159°C, et le taux de reflux vaut 1.